# EUROPEAN PATENT SPECIFICATION

(11) **EP 1 854 342 B1**
(45) Date of publication and mention of the grant of the patent: **11.06.2014**
(21) Application number: 06704928.8
(22) Date of filing: 28.02.2006
(51) Int. Cl.: H05K 1/00, A61B 5/00, G08B 21/00, A61F 13/15, G08B 23/00

(54) **FLEXIBLE ELECTRONIC DEVICE**
FLEXIBLE ELEKTRONISCHE EINRICHTUNG
DISPOSITIF ELECTRONIQUE FLEXIBLE

(30) Priority: 28.02.2005 AU 2005900931
(43) Date of publication of application: 14.11.2007
(60) Divisional application: 14164439.3
(73) Proprietor: Commonwealth Scientific and Industrial Research Organization, Campbell, Australian Capital Territory 2612 (AU)
(72) Inventor: MESTROVIC, Michael, Moriac, Victoria 3240 (AU); HUMPHRIES, Bill, Newtown, Victoria 3220 (AU); D'ARCY, Brendan, Grovedale, Victoria 3216 (AU); HELMER, Richard, Geelong West, Victoria 3218 (AU)
(74) Representative: Finnie, Peter John
(86) International application number: PCT/AU2006/000253
(87) International publication number: WO 2006/089377

(56) References cited:
- WO-A1-93/10828
- WO-A1-99/56613
- WO-A1-2005/107580
- US-A- 4 730 625
- US-A- 5 264 830
- US-A- 5 838 240
- US-A1- 2002 145 525
- US-A1- 2004 220 538
- US-A1- 2004 220 538
- US-B1- 6 246 330

## Description

### FIELD AND BACKGROUND OF THE INVENTION

The present invention relates to a device that may be worn by a person or fitted to an apparatus and includes any one or a combination of the following: a sensor for sensing or measuring a parameter, a control interface for controlling a parameter, or a monitoring interface for monitoring a parameter. The parameter may be any condition, property or state including environmental or operating parameters, or vital signs of life. In addition, the parameter may be any operating parameter of an electronic device.

For example, in the situation where the device of the present invention is in the form of a disposable diaper or nappy, the device may include a sensor adapted for signalling when the nappy or diaper requires changing.

According to another example, the device of the present invention may be in the form of a singlet, vest, or T-shirt including a sensor that is adapted for monitoring the heart rate and/or respiration rate of a patient, athlete, or person wearing the device.

According to another example, the device of the present invention may be in the form of a textile including a control interface with single or multiple switches or buttons that control the function of an electronic device. In the case where the electronic device is in the form of a radio or media player, the control interface may be used for controlling an operating parameter such as the volume of sound played by the device.

According to yet another example, the device of the present invention may be in the form of a belt including a sensor that can be fitted to an apparatus. The sensor may be adapted for use as either an impact or strain gauge for evaluating the forces applied to the apparatus or recording the amount of work performed by the apparatus.

There are a large number of devices presently available that are capable of sensing, controlling, or monitoring various parameters such a moisture, strain, and heart rate or the operating parameter of an electronic device. However, in most cases the cost of manufacture of the devices requires that the devices be reused a number of times.

US 2002/0145525 A1 discloses a patient fluid discharge monitoring method and apparatus that includes at least one article configured to be worn by a patient, the article having absorbent material and a RF tag received adjacent the absorbent material. The RF tag is excited with an excitation signal and the response of the RF tag to the excitation signal is detected. The detected response of the RF tag is compared to a predetermined response. The RF tag has a first detected response when the absorbent material has no fluid therein and a second detected response when the absorbent material has fluid therein.

US 6,246,330 B1 discloses an elimination-absorber monitoring system that addresses diaper-monitoring problems with a unique, low cost, multi-layer disposable sensor structure that absorbs small volumes of urine, yet allows most urine volume to flow unimpeded through it, and into the diaper below. When connected with a reusable, miniature monitor/indicator unit, the sensor presents a clear and on-going change of measurement condition upon experiencing a rapid influx into the diaper of a significant volume of urine, and/or upon a significant reduction in the available absorbency of the diaper's top surface. The sensor additionally provides recessed, protected elements for similarly presenting a clear and on-going change in measurement condition upon experiencing the presence of fecal matter. Further provided is the monitor unit employing narrow, widely-spaced, fast rise-time, fast transition-time pulses for conductivity measurement and alarm activation. The monitor and sensor are interconnected and attached to a diaper by particularly effective and unique means, and the monitor is equipped with a highly intuitive and convenient control interface, as well as improved assemblies for the transmission of audible and visual alarm indications. Also described is a convenient test-strip device which, when connected to the monitor/alarm unit of the system, can selectively simulate either a soiled or unsoiled elimination-absorber/sensor for test, caregiver-training or demonstration purposes.

### SUMMARY OF THE INVENTION

According to a first aspect of the invention, there is provided a device as set out in Claim 1.

According to a second aspect of the invention, there is provided a device as set out in Claim 24.

An advantage provided by the present invention is that it enables the interchangeability, assembly and re- assembly of the first and second parts of the electrical circuit in a "transient" manner. In particular, an advantage provide by the first and second parts of the circuit being disconnectable is that when the flexible substrate requires disposal or maintenance such as washing, the second part can be disconnected and reused once the flexible substrate has been substituted with a fresh substrate. The electronic components included in the first and second parts of the circuit can be configured to suit particular applications and, therefore, whether the flexible substrate will be washed or disposed.

Throughout this specification the terms "flexible" or "flexible substrate" embrace any material that can change in shape or configuration. It is ideal, but by no means mandatory or compulsory that the change in shape or configuration can be the result of incidental forces applied to the material and that the changes in shape and configuration of the material are not permanent or irreversible. Examples of flexible substrates include but are by no means limited to: textile material and non-textile material such as plastic and board material that can flex without having drape properties traditionally possessed by textiles.

Although it is possible that the first part of the circuit may be indirectly attached to flexible substrate, it is preferred that the first part of the circuit be directly attached to the flexible substrate.

It is preferred that the first part of the circuit be able to flex without retarding the flexibility of the flexible substrate.

It is also preferred that the first part of the circuit be formed on or embedded in the flexible substrate using electronic circuit printing techniques and or textile processing techniques which enable the incorporation of conductive materials, such as conductive filaments and yarns, by knitting, weaving, and layering, and/or combinations of these techniques.

It is possible that the device may also include a second substrate, preferably in the form of a second flexible substrate and that the second part of the circuit be carried by the second flexible substrate. During use, the first or second substrate may require washing or substitution with a fresh substrate. For example, in the situation where the first substrate is due for washing or disposal, it is envisaged that the second part of the circuit will include the more valuable component of the electrical circuit.

It is preferred that the device be in the form of a textile and that the flexible substrate on which the first part of the circuit is carried be incorporated in the textile. In this situation and when the first part of the circuit includes a sensing element and/or control interface, an advantage provided by flexible substrate being incorporated into a textile or garment is that the sensing element can sense parameters very close to a person's skin such as surface temperature, skin conductivity and moisture and/or provide an intimate monitor interface and or control interface for controlling electronic devices.

The term "textile or garment" embraces any form of garment that may be worn by a person including jumpers, sweaters, vests, pants, T-shirts, underwear, belts, gowns, personal flotation devices, safety apparel, nappies and diapers, and any other form of textile such as that used during road construction, sun shade or solar protection, and wrapping.

Although it is possible that the first and second parts can be held together in electrical connection by any suitable means including clips, clasps, Velcro™ and other co-operating male and female members, it is preferred that a releasable conductive adhesive bond the first and second parts of the circuit together.

The adhesive may also be used with one or a combination of the other types of mechanisms mentioned in the preceding paragraph for holding the first and second parts together.

Any suitable adhesive that resists accidental separation of the first and second parts of the circuit yet allows the first and second parts to be manually separated would be suitable. In other words, according to one preferred form of the invention, it is envisaged that the adhesive provide a basis for a transient electrical connection between the first and second parts of the circuit.

It is preferred that the first part of the circuit include a first pair of contact surfaces and that the second part of the circuit include a second pair of co-operating contact surfaces, and that the first and second pairs of the contact surfaces can be manually aligned and placed together to facilitate electrical connection therebetween. It is also preferred that the first and second parts be able to be manually disconnected so that either one or both of the parts can be reused.

In the situation where an adhesive is at least in part responsible for holding the first and second parts together in electrical connection, it is preferred that the adhesive be located between the first and second pairs of the contact surfaces and that the adhesive preferably be an electrically conductive adhesive so that an electrical path can be established between the first and second pairs of contact surfaces.

It is preferred that the electrical contact surface of each of the first or second part have a total surface area of greater than or equal to 1 square millimetre.

In the situation where an adhesive is located between the first and second pairs of contact surfaces when the electrical connection is made, it is preferred that the adhesive be pre-applied to one of the contact surfaces before electrical connection and that a removable protective tab be positioned to cover the adhesive on the contact surfaces and prevent the adhesive from bonding to other objects. In use, the protective tab can be removed just prior to the first and seconds part being placed together to form the electrical connection and can be replaced over the contact surfaces when the parts have been separated after use.

The electrical circuit may have any required electronic components including but not limited to:
- an electrical power source for powering the circuit;
- a processing unit for receiving information from the controller, monitor or sensor on the parameter being monitored, sensed or controlled and processing the information using suitable techniques, such as by comparing the information on the parameter to a pre-selected value or set of values of the parameter;

- a controller interface for allowing the on and off operation of the power source or control another operational function of the device;
- an output system for sending processed and/or unprocessed signals and/or for signalling when information on the parameter being controlled, monitored or sensed equals, exceeds or is less than a pre-selected value of the parameter being monitored, sensed or controlled.

Preferably, the transmitter includes a suitable antenna for transmitting a signal to a remote receiver that informs a person that a selected condition or parameter change has occurred and that action may be required. For example, in the situation where the garment is a disposable diaper and the electrical circuit has the capacity to transmit a signal to a centralised care station or care manager who may be caring for a number of persons, the device can then be used to identify when and on which persons need their nappies changed.

In the situation in which the flexible substrate forms part of a disposable diaper, pad, incontinency sanitary product or wound dressing, and the parameter being monitored, sensed or controlled is moisture and the first part of the circuit carried by the flexible substrate forms part of the garment and can be disposed and the second part of the circuit can be reused by removing it from the used garment and connecting it to a fresh garment. In order to reduce overall costs, it is envisaged that the second part which to be reused would be designed to include the more expensive components of the electrical circuit.

In the situation when the device is worn by a person and includes a sensor, the sensor may be adapted for sensing parameters such as, but by no means limited to, surface temperature, heart rate or respiration rate. In the situation when the device worn by a person includes a control interface in the form of one or more switches, the switches may form part of a media player and be used for controlling the volume or selection of music played.

In the situation where in the device is fitted to an apparatus or placed on an object, the sensor may sense one or more parameters such as but by no means limited to strain, temperature, moisture, light intensity, sound level, pressure and impact forces.

### BRIEF DESCRIPTION OF THE DRAWING

Two preferred embodiments of the present invention will now be described with reference to the accompanying drawings, of which:
Figures 1 and 2 illustrate perspective views of flexible and rigid substrates respectively according to an embodiment of a device of the present invention wherein the substrates are disconnected;
Figure 3 illustrates a perspective of the flexible and rigid substrates shown in Figures 1 and 2 connected together;
Figures 4 and 5 illustrates perspective views of two flexible substrates that are disconnected according to an alternative embodiment of a device of the present invention; and
Figure 6 illustrates a perspective view of the two flexible substrates shown in Figures 4 and 5 connected together.

### DETAILED DESCRIPTION

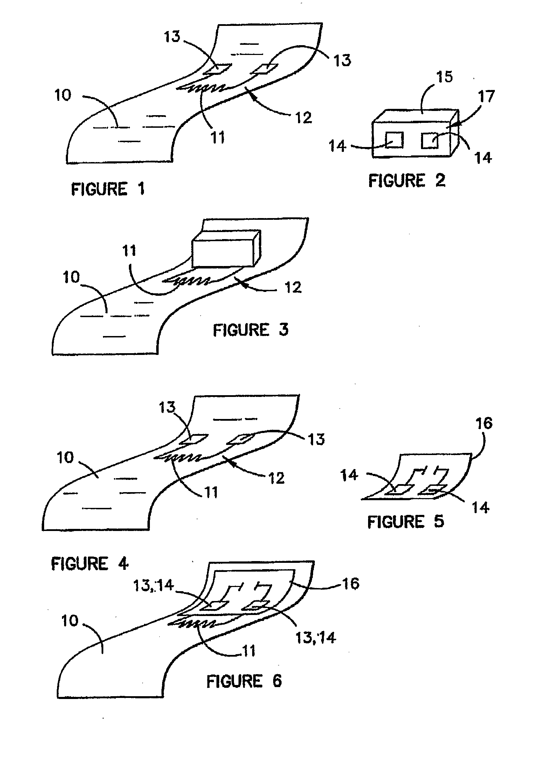

The embodiments of the invention shown in Figures 1 to 6 include a number of features in common and the same reference numerals have been used to identify the same or similar features on both embodiments.

The embodiments of the invention shown in the Figures are described in the context of sensing moisture in disposable diapers, pads and alike sanitary products that are disposed or destroyed after use. However, it will be appreciated that the present invention is able to be used in the wide range of other applications such as protective or conventional clothing and covers that can be fitted to apparatuses. In the situation where the device of the invention forms part of a garment such as pants, a jacket or a shirt, it is envisaged that the device include electrical circuitry having at least two parts and that at least one part of the circuit is mounted on or in embedded in the textile material that makes up the garment. When the garment requires washing, the electrical circuit can be separated into parts such that only the washable component remains on the garment during washing. The electrical circuit may be adapted for sensing, monitoring or controlling parameters such as temperature, strain, movement of the garment or the operation of a device.

With reference to the embodiment of the invention shown in Figures 1 to 3, the device includes a flexible substrate identified by reference numeral 10 that forms part of a diaper and on which a first part 12 of an electrical circuit is embedded and/or printed using suitable techniques and technology. The first part 12 of the electrical circuit shown in Figure 1 includes a conventional moisture sensor 11 for sensing bodily excretion indicating when the diaper needs to be changed.

The flexible substrate 10 may be any suitable material such as the liquid permeable material commonly used to manufacture diapers. It will be appreciated that the first part 12 of the electrical circuit need not be directly attached to the flexible substrate as shown in the Figures. For example, it is possible that the first part 12 of the electrical circuit carried by the flexible substrate may be directly attached to a rigid sheet which is bonded or laminated to the flexible substrate 10. However, to avoid the device from impeding the person's movement or reducing the comfort of the garment, it is preferred that the first part 12 of the circuit be directly attached or embedded in the flexible substrate 10 and not retard the flexibility of the substrate 10.

The first part 12 of the electrical circuit also includes two contact surfaces 13 in the form of electrodes for forming an electrical connection with corresponding electrodes 14 formed on the underside of the rigid substrate 15 shown in Figure 2. The rigid substrate 15 houses the remaining components of a second part 17 the electrical circuit including a power source, logic chip and output transmitter for sending signals to a receiver or a station of receivers operated by a manager caring for a number of patients. The first and second parts 12 and 17 of the electrical circuit form an operable circuit for sensing, control or monitoring a parameter.

Although not shown in the Figures, an electrical conductive adhesive is spread over the electrodes 13 and 14 of either one or a combination of the rigid or flexible substrates 10 and 15. The adhesive may also be located on the regions of the flexible substrate 10 adjacent to the electrodes and on the underside of the rigid substrate 15. In addition, a protective cover not shown in the Figures may be placed over the adhesive on either one or both of the substrates 10 and 15 to prevent removal of adhesive and prevent contamination of the adhesive by unwanted particles sticking to the adhesive.

An example of a suitable conductive adhesive is electrical conductive tape sold under the trade name ARclad™ 8001 by Adhesive Research, Inc. Glen Rock PA 17327.

In use, the protective cover is removed from the flexible and rigid substrates 10 and 15 to expose the electrodes 13 and 14. The rigid substrate 15 is then manually placed on top of the flexible substrate 10 with the pairs of electrodes 13 and 14 in alignment. The adhesive between the substrates 10 and 15 and electrodes 13 and 14 will bond the two substrates 10 and 15 together and is of sufficient strength to prevent the substrates from inadvertently coming apart during use. However, the strength of the adhesive is not so strong so as to prevent the flexible and rigid substrates 10 and 15 from being manually separated without damaging the electrodes 13 and 14.

Once the flexible substrate or diaper to which it is fixed has been soiled, it is envisaged that the second part of the circuit, housed by the rigid substrate 15 is able to be separated or removed from the flexible substrate 10 so that the rigid housing can be reused by being fitted to a fresh diaper. The flexible substrate 10 forming part of a diaper and first part 12 of the circuit can be disposed or washed. In order to reduce costs, it is envisaged that the second part 17 of the circuit that is intended to be reused includes the more costly components of the electric circuit.

The second embodiment shown in the Figures 4 to 6 is substantially the same as the embodiment shown in Figures 1 to 3. The only substantial difference between the embodiments is that the rigid substrate 15 of the embodiment shown in figures 1 to 3 has been substituted with a flexible substrate 16 which like the rigid substrate 15 of the first embodiment includes all of the components of the circuit that are intended to be reused. The substrates 10 and 16 are also bonded together with the pairs of contact surfaces 13 and 14 in electrical connection using a suitable adhesive.

A person skilled in the art of the present invention will appreciate that many modifications and variations may be made to the preferred embodiments described above without departing from the scope of the present invention as defined in the appended claims.

For example, the first part of the circuit mounted or embedded in the flexible substrate may include, but is by no means limited to, any one or combination of the following electrical components:
- a thermocouple for sensing temperature;
- a strain gauge for monitoring movement or impact forces;
- a light or sound sensor;
- a switch or any other form of control interface that allows adjustment; and
- suitable couplings for sensing heart rate or respiration rate of patient or athlete.

## Claims

1. A device that may be worn by a person or fitted to an apparatus for monitoring, sensing or controlling one or more parameters such as, but by no means limited to, moisture, temperature, heart rate, respiration rate, strain, movement, light intensity, sound, pressure or impact forces, or an electrical function, wherein the device includes:
a) a flexible substrate (10) that can be worn by a person or attached to an apparatus, wherein the flexible substrate requires disposal or replacement with a fresh flexible substrate or requires maintenance that prevents continuous use of the flexible substrate; and
b) an electrical circuit having separate first and second parts (12, 16) and one or both of the first and second parts includes any one or a combination of a controller for controlling a parameter, a monitor (11) for monitoring a parameter or a sensor for sensing a parameter, wherein the first part (12) is carried by the flexible substrate (10) and includes a first pair of contact surfaces (13) and the second part includes a second pair of contact surfaces (14);
**characterised in that** an electrical conductive adhesive is present on the contact surfaces of either one or both of the first and second pairs (13, 14) and electrical current can be conducted therethrough when the first and second pairs are connected together to form an electrical connection therebetween and thereafter disconnected to allow the parts to be reused or disposed as desired.

2. The device according to claim 1, wherein the first part of the circuit is directly attached to the flexible substrate.

3. The device according to claim 1 or 2, wherein the first part of the circuit is able to flex such that both the flexible substrate and the first part of the circuit is able to be flexed.

4. The device according to any one of claims 1 to 3, wherein the first part of the circuit includes printed circuitry that is formed on or embedded in the flexible substrate.

5. The device according to any one of claims 1 to 4, wherein the first part of the circuit includes an electrical conductive material that is on or embedded in the flexible substrate.

6. The device according to claim 5, wherein the conductive material is a filament or yarn that has been woven, knitted or incorporated in the flexible substrate by way of a layered structure.

7. The device according to ay one of claims 1 to 6, wherein the second part of the circuit is directly or indirectly connected to a second flexible substrate.

8. The device according to any one of claims 1 to 7, wherein the second part of the circuit is housed by a rigid enclosure.

9. The device according to any one of claims 1 to 7, wherein the second part of the circuit is carried or supported on another flexible substrate.

10. The device according to any one of claims 1 to 9, wherein the first and second parts are able to be manually connected and disconnected.

11. The device according to claim 10, wherein the first and second parts are, in addition to the conductive adhesive, held together in electrical connection by any one or a combination of clips, clasps, Velcro™, any other co-operating male and female members.

12. The device according to claim 11, wherein the adhesive resists accidental separation of the first and second parts of the circuit yet allows the first and second parts to be manually separated.

13. The device according to any one of claims 1 to 12, wherein, the first and second pairs of the contact surfaces can be manually aligned and placed together to facilitate electrical connection therebetween.

14. The device according to claim 13, wherein an electrical conductive adhesive is pre-applied to either one or both of the first and second pairs of contact surfaces.

15. The device according to claim 14, wherein a removable protective tab is positioned to cover the adhesive on the contact surfaces so as to prevent contamination thereof prior to use.

16. The device according to any one of claims 13 to 15, wherein the contact surfaces of the first or second parts have a total surface area of greater than or equal to 1 square millimetre.

17. The device according to any one of claims 1 to 16, wherein the electrical circuit includes any one or a combination of:
• an electrical power source for powering the circuit;
• a processing unit for receiving information from the controller, monitor or sensor on the parameter being monitored, sensed or controlled and for comparing the information on the parameter to a pre-selected value or set of values of the parameter;
• a controller interface for allowing the on and off operation of the power source or control another operational function of the device;
• an output system for sending processed or unprocessed signals or for signalling when information on the parameter being controlled, monitored or sensed equals, exceeds or is less than a pre-selected value of the parameter being monitored, sensed or controlled.

18. The device according to claim 17, wherein the processing unit is a processing chip or micro controller.

19. The device according to claim 17, wherein the control interface is a switch.

20. The device according to any one of claims 17 to 19, wherein the output system includes either one or combination of: a transmitter that transmits a signal to a receiver; or an indicator that emits light and/or sound.

21. The device according to any one of claims 18 to 20, wherein the output system transmits a signal to a centralized station or care manager who may be caring for one or any number of patients.

22. The device according to any one of claims 1 to 19, wherein the flexible substrate on which the first part of the circuit is carried is incorporated in, or forms part of, a garment or a textile for making a garment.

23. The device according to claim 22, wherein the garment is a disposable diaper, pad, incontinency sanitary product or wound dressing, and the parameter being monitored, sensed or controlled is moisture and the first part of the circuit carried by the flexible substrate forms part of the garment and can be disposed and the second part of the circuit can be reused by removing it from the used garment and connecting it to a fresh garment.

24. A device that may be worn by a person or fitted to an apparatus for monitoring, sensing or controlling one or more parameters such as, but by no means limited to, moisture, temperature, heart rate, respiration rate, strain; movement, light intensity, sound, pressure or impact forces, or an electrical function, wherein the device includes:
a) a flexible substrate (10) that can be worn by a person or attached to an apparatus, wherein the flexible substrate (10) requires disposal or replacement with a fresh flexible substrate or requires maintenance that prevents continuous use of the flexible substrate (10); and
b) a first part (12) of an electrical circuit that is connectable to a second part (16) of the electrical circuit, wherein the first part (12) is attached to the flexible substrate (10) and the first part (12) includes a pair of contact surfaces (13) for connection with a co-operating pair of contact surfaces (14) of the second part (16);
**characterised in that** an electrical conductive adhesive is pre-applied to the contact surfaces (13) of the first part (12) such that the first and second parts (12; 16) can be connected together to form an electrical connection therebetween and thereafter disconnected to allow the first part of the circuit to be disposed as desired together with the substrate.

25. The device according to claim 24, wherein the first part of the circuit is able to flex such that both the flexible substrate and the first part of the circuit is able to be flexed.

26. The device according to claim 24 or 25, wherein the first part of the circuit includes printed circuitry that is formed on or embedded in the flexible substrate.

27. The device according to any one of claims 24 to 26, wherein the first part of the circuit includes an electrical conductive material that is on or embedded in the flexible substrate.

28. The device according to claim 26, wherein the conductive material is a filament or yarn that has been woven, knitted or incorporated in the flexible substrate by way of a layered structure.

29. The device according to any one of claims 24 to 28, wherein prior to connection of the first and second parts a removable protective tab is positioned to cover the adhesive on the contact surfaces so as to prevent contamination thereof prior to use.

30. The device according to any one of claims 24 to 29, wherein the contact surfaces of the first part have a total surface area of greater than or equal to 1 square millimetre.

31. The device according to any one of claims 24 to 30, wherein the flexible substrate on which the first part of the circuit is attached is incorporated in, or forms part of, a garment or a textile for making a garment.

32. The device according to claim 31, wherein the garment is a disposable diaper, pad, incontinency sanitary product or wound dressing, and the parameter being monitored, sensed or controlled is moisture and the first part of the circuit carried by the flexible substrate forms part of the garment and can be disposed and the second part of the circuit can be reused by removing it from the used garment and connecting it to a fresh garment.

## Patentansprüche

1. Einrichtung, die von einer Person getragen oder an eine Vorrichtung zum Überwachen, Abtasten oder Steuern eines oder mehrerer Parameter, wie beispielsweise, aber nicht darauf beschränkt, von Feuchte, Temperatur, Herzfrequenz, Atemfrequenz, Anstrengung, Bewegung, Lichtintensität, Schall, Druck oder Aufprallkräfte oder elektrische Funktion angebracht werden könnte, wobei die Einrichtung einschließt:
a) ein flexibles Substrat (10), das von einer Person getragen oder an eine Vorrichtung angebracht werden kann, wobei das flexible Substrat Entsorgung oder Ersatz mit einem frischen Substrat erforderlich macht oder Wartung erfordert, die kontinuierliche Verwendung des flexiblen Substrats verhindert; und
b) einen elektrischen Schaltkreis mit separaten ersten und zweiten Teilen (12, 16) und wobei einer oder beide der ersten und zweiten Teile einen beliebigen oder eine Kombination von einem Controller zum Steuern eines Parameters, einen Monitor (11) zum Überwachen eines Parameters oder einen Sensor zum Abtasten eines Parameters einschließt, wobei das erste Teil (12) vom flexiblen Substrat (10) getragen wird und ein erstes Paar von Kontaktoberflächen (13) einschließt und das zweite Teil ein zweites Paar von Kontaktoberflächen (14) einschließt;
**dadurch gekennzeichnet, dass** ein elektrisch leitender Klebstoff auf den Kontaktoberflächen entweder eines oder beider der ersten und zweiten Teile (13, 14) zugegen ist und elektrischer Strom dort hindurch geleitet werden kann, wenn die ersten und zweiten Teile zusammengeschlossen sind, um eine elektrische Verbindung dazwischen zu bilden und danach getrennt werden, damit die Teile wie erwünscht wiederverwendet oder entsorgt werden können.

2. Einrichtung nach Anspruch 1, wobei das erste Teil des Schaltkreises direkt an das flexible Substrat angebracht ist.

3. Einrichtung nach Anspruch 1 oder 2, wobei das erste Teil des Schaltkreises biegsam ist, derartig, dass sowohl das flexible Substrat als auch das erste Teil des Schaltkreises gebeugt werden kann.

4. Einrichtung nach einem beliebigen der Ansprüche 1 bis 3, wobei das erste Teil des Schaltkreises gedruckte Schaltung einschließt, die auf dem flexiblen Substrat gebildet oder in dieses eingebettet ist.

5. Einrichtung nach einem beliebigen der Ansprüche 1 bis 4, wobei das erste Teil des Schaltkreises ein elektrisch leitendes Material einschließt, das auf dem flexiblen Substrat gebildet oder in dieses eingebettet ist.

6. Einrichtung nach Anspruch 5, wobei das leitende Material ein Filament oder Garn ist, das gewebt, gestrickt oder mittels einer geschichteten Struktur in das flexible Substrat eingebaut worden ist.

7. Einrichtung nach einem beliebigen der Ansprüche 1 bis 6, wobei das zweite Teil des Schaltkreises direkt oder indirekt an ein zweites flexibles Substrat angeschlossen ist.

8. Einrichtung nach einem beliebigen der Ansprüche 1 bis 7, wobei das zweite Teil des Schaltkreises in einem starren Gehäuse untergebracht ist.

9. Einrichtung nach einem beliebigen der Ansprüche 1 bis 7, wobei das zweite Teil des Schaltkreises auf einem anderen flexiblen Substrat getragen oder gestützt wird.

10. Einrichtung nach einem der Ansprüche 1 bis 9, wobei die ersten und zweiten Teile manuell verbunden oder getrennt werden können.

11. Einrichtung nach Anspruch 10, wobei die ersten und zweiten Teile, zusätzlich zum leitenden Klebstoff, durch einen beliebigen oder eine Kombination von Klemmen, Schnallen, Velcro™, irgendwelchen anderen kooperierenden männlichen und weiblichen Elementen in elektrischer Verbindung zusammengehalten werden.

12. Einrichtung nach Anspruch 11, wobei der Klebstoff zufälliger Trennung der ersten und zweiten Teile des Schaltkreises entgegenwirkt, dennoch manuelle Trennung der ersten und zweiten Teile zulässt.

13. Einrichtung nach einem beliebigen der Ansprüche 1 bis 12, wobei die ersten und zweiten Paare der Kontaktoberflächen manuell ausgerichtet und zusammen platziert werden, um elektrische Verbindung dazwischen zu erleichtern.

14. Einrichtung nach Anspruch 13, wobei ein elektrisch leitender Klebstoff auf entweder eine oder beide der ersten und zweiten Paare von Kontaktflächen vorbeschichtet wird.

15. Einrichtung nach Anspruch 14, wobei eine abnehmbare Schutzlasche positioniert ist, den Klebstoff auf den Kontaktflächen abzudecken, um deren Kontamination vor Gebrauch zu verhindern.

16. Einrichtung nach einem beliebigen der Ansprüche 13 to 15, wobei die Kontaktoberflächen der ersten und zweiten Teile eine gesamte Oberfläche größer als oder gleich 1 Quadratmillimeter haben.

17. Einrichtung nach einem beliebigen der Ansprüche 1 bis 16, wobei der elektrische Schaltkreis irgendeine oder eine Kombination des Folgenden einschließt:
• eine elektrische Stromquelle um den Schaltkreis mit Strom zu versorgen;
• eine Zentraleinheit zum Empfangen von Information vom Controller, Monitor oder Sensor über den Parameter, der überwacht, abgetastet oder gesteuert wird und zum Vergleichen der Information über den Parameter mit einem vorgewählten Wert oder Satz von Werten des Parameters;
• eine Controllerschnittstelle, die den Betrieb Ein/Aus der Stromquelle oder die Steuerung einer weiteren Betriebsfunktion der Einrichtung zulässt;
• ein Ausgabesystem zum Senden verarbeiteter oder unverarbeiteter Signale oder zum Signalisieren, wenn Information über den Parameter, der gesteuert, überwacht oder abgetastet wir, einen vorgewählten Wert des überwachten, abgetasteten oder gesteuerten Parameters überschreitet oder geringer ist.

18. Einrichtung nach Anspruch 17, wobei die Zentraleinheit ein Prozessorchip oder Mikrocontroller ist.

19. Einrichtung nach Anspruch 17, wobei die Steuerschnittstelle ein Schalter ist.

20. Einrichtung nach einem beliebigen der Ansprüche 17 bis 19, wobei das Ausgabesystem entweder eins oder eine Kombination des Folgenden einschließt: einen Transmitter, der ein Signal an einen Empfänger sendet; oder einen Anzeiger, der Licht und/oder Schall emittiert.

21. Einrichtung nach einem beliebigen der Ansprüche 18 bis 20, wobei das Ausgabesystem ein Signal an eine zentralisierte Station oder an einen Pflegemanager, der sich möglicherweise um einen oder eine Anzahl von Patienten kümmert.

22. Einrichtung nach einem beliebigen der Ansprüche 1 bis 19, wobei das flexible Substrat, das den ersten Teil des Schaltkreises trägt, in ein Kleidungsstück eingebaut ist oder einen Teil davon bildet oder ein Textil zur Herstellung eines Kleidungsstücks ist.

23. Einrichtung nach Anspruch 22, wobei das Kleidungsstück eine Einwegwindel, ein Pad, ein Sanitärprodukt für Inkontinenz oder Wundverband ist und der zu überwachende, abzutastende oder zu steuernde Parameter Feuchte ist und das erste Teil des Schaltkreises, der vom flexiblen Substrat getragen wird, bildet Teil des Kleidungsstücks und kann entsorgt werden und das zweite Teil des Schaltkreises lässt sich wiederverwenden, indem es vom gebrauchten Kleidungsstück entfernt und an ein frisches Kleidungsstück angeschlossen wird.

24. Einrichtung, die von einer Person getragen oder an eine Vorrichtung zum Überwachen, Abtasten oder Steuern eines oder mehrerer Parameter, wie beispielsweise, aber nicht darauf beschränkt, von Feuchte, Temperatur, Herzfrequenz, Atemfrequenz, Anstrengung, Bewegung, Lichtintensität, Schall, Druck oder Aufprallkräfte oder eine elektrische Funktion angebracht werden könnte, wobei die Einrichtung einschließt:
a) ein flexibles Substrat (10), das von einer Person getragen oder an eine Vorrichtung befestigt werden kann, wobei das flexible Substrat (10) Entsorgung oder Ersatz mit einem frischen Substrat erforderlich macht oder Wartung erfordert, die kontinuierliche Verwendung des flexiblen Substrats (10) verhindert; und
b) ein erstes Teil (12) eines elektrischen Schaltkreises, das an das zweite Teil (16) des elektrischen Schaltkreises anschließbar ist, wobei das erste Teil (12) am flexiblen Substrat (10) befestigt ist und das erste Teil (12) ein Paar von Kontaktoberflächen (13) zur Verbindung mit einem kooperierenden Paar von Kontaktoberflächen (14) des zweiten Teils (16) einschließt;
**dadurch gekennzeichnet, dass** ein elektrisch leitender Klebstoff auf die Kontaktoberflächen (13) des ersten Teils (12) derartig vorbeschichtet ist, dass sich die ersten und zweiten Teile (12, 16) zusammenschließen lassen, um eine elektrische Verbindung dazwischen zu bilden und danach getrennt werden können, um zu erlauben, dass das erste Teil des Schaltkreises wie erwünscht zusammen mit dem Substrat entsorgt werden kann.

25. Einrichtung nach Anspruch 24, wobei das erste Teil des Schaltkreises biegsam ist, derartig, dass sowohl das flexible Substrat als auch das erste Teil des Schaltkreises gebeugt werden kann.

26. Einrichtung nach Anspruch 24 oder 25, wobei das erste Teil des Schaltkreises gedruckte Schaltung einschließt, die auf dem flexiblen Substrat gebildet oder in dieses eingebettet ist.

27. Einrichtung nach einem beliebigen der Ansprüche 24 bis 26, wobei das erste Teil des Schaltkreises ein elektrisch leitendes Material einschließt, das auf dem flexiblen Substrat gebildet oder in dieses eingebettet ist.

28. Einrichtung nach Anspruch 26, wobei das leitende Material ein Filament oder Garn ist, das gewebt, gestrickt oder mittels einer geschichteten Struktur in das flexible Substrat eingebaut worden ist.

29. Einrichtung nach einem beliebigen der Ansprüche 24 bis 28, wobei vor Verbinden der ersten und zweiten Teile eine abnehmbare Schutzlasche positioniert wird, um den Klebstoff auf den Kontaktoberflächen abzudecken, um deren Kontamination vor Verwendung zu verhindern.

30. Einrichtung nach einem beliebigen der Ansprüche 24 bis 29, wobei die Kontaktoberflächen des ersten Teils eine gesamte Oberfläche größer als oder gleich 1 Quadratmillimeter haben.

31. Einrichtung nach einem beliebigen der Ansprüche 24 bis 30, wobei das flexible Substrat, auf dem das erste Teil des Schaltkreises befestigt ist, in ein Kleidungsstück eingebaut ist oder einen Teil davon bildet oder ein Textil zur Herstellung eines Kleidungsstücks ist.

32. Einrichtung nach Anspruch 31, wobei das Kleidungsstück eine Einwegwindel, ein Pad, ein Sanitärprodukt für Inkontinenz oder Wundverband ist und der zu überwachende, abzutastende oder zu steuernde Parameter Feuchte ist und das erste Teil des Schaltkreises, der vom flexiblen Substrat getragen wird, bildet Teil des Kleidungsstücks und kann entsorgt werden und das zweite Teil des Schaltkreises lässt sich wiederverwenden, indem es vom gebrauchten Kleidungsstück entfernt und an ein frisches Kleidungsstück angeschlossen wird.

## Revendications

1. Dispositif qui peut être porté par une personne ou installé sur un appareil à des fins de surveillance, de détection ou de commande d'un ou de plusieurs paramètres tels, mais de façon non limitative, l'humidité, la température, le rythme cardiaque, le rythme respiratoire, l'effort, le mouvement, l'intensité lumineuse, le son, les forces de pression ou d'impact, ou une fonction électrique, dans lequel le dispositif comprend :
a) un substrat flexible (10) qui peut être porté par une personne ou être attaché sur un appareil, dans lequel le substrat flexible a besoin d'être jeté ou remplacé par un nouveau substrat flexible ou a besoin d'un entretien qui empêche une utilisation continue du substrat flexible ; et
b) un circuit électrique ayant des première et seconde parties séparées (12, 16), et l'une ou les deux parmi les première et seconde parties comprenant l'un quelconque ou une combinaison parmi un dispositif de commande à des fins de commande d'un paramètre, un dispositif de surveillance (11) à des fins de surveillance d'un paramètre ou un dispositif de détection à des fins de détection d'un paramètre, dans lequel la première partie (12) est portée par le substrat flexible (10) et comprend une première paire de surfaces de contact (13) et la seconde partie comprend une seconde paire de surfaces de contact (14) ;
**caractérisé en ce qu'**un adhésif conducteur est présent sur les surfaces de contact de l'une ou des deux parmi les première et seconde paires (13, 14) et un courant électrique peut être conduit au travers de celles-ci quand les première et seconde paires sont connectées ensemble pour former une connexion électrique entre elles et par la suite déconnectées pour permettre aux parties d'être réutilisées ou jetées selon les besoins.

2. Dispositif selon la revendication 1, dans lequel la première partie du circuit est directement attachée au substrat flexible.

3. Dispositif selon la revendication 1 ou la revendication 2, dans lequel la première partie du circuit est en mesure de fléchir de telle sorte qu'à la fois le substrat flexible et la première partie du circuit sont en mesure d'être fléchis.

4. Dispositif selon l'une quelconque des revendications 1 à 3, dans lequel la première partie du circuit comprend un circuit imprimé qui est formé sur le substrat flexible ou qui est intégré dans celui-ci.

5. Dispositif selon l'une quelconque des revendications 1 à 4, dans lequel la première partie du circuit comprend un matériau conducteur qui est sur le substrat flexible ou qui est intégré dans celui-ci.

6. Dispositif selon la revendication 5, dans lequel le matériau conducteur est un filament ou fil qui a été tissé, tricoté ou incorporé dans le substrat flexible au moyen d'une structure en couches.

7. Dispositif selon l'une quelconque des revendications 1 à 6, dans lequel la seconde partie du circuit est connectée directement ou indirectement à un second substrat flexible.

8. Dispositif selon l'une quelconque des revendications 1 à 7, dans lequel la seconde partie du circuit est logée dans un boîtier rigide.

9. Dispositif selon l'une quelconque des revendications 1 à 7, dans lequel la seconde partie du circuit est portée ou supportée sur un autre substrat flexible.

10. Dispositif selon l'une quelconque des revendications 1 à 9, dans lequel les première et seconde parties sont en mesure d'être connectées et déconnectées manuellement.

11. Dispositif selon la revendication 10, dans lequel les première et seconde parties sont, en plus de l'adhésif conducteur, retenues ensemble en une connexion électrique par l'un quelconque ou par une combinaison parmi des clips, des agrafes, du Velcro™, et autres éléments mâles et femelles qui coopèrent.

12. Dispositif selon la revendication 11, dans lequel l'adhésif résiste à toute séparation accidentelle des première et seconde parties du circuit et permet cependant aux première et seconde parties d'être séparées manuellement.

13. Dispositif selon l'une quelconque des revendications 1 à 12, dans lequel les première et seconde paires de surfaces de contact peuvent être manuellement alignées et placées ensemble pour faciliter la connexion électrique entre celles-ci.

14. Dispositif selon la revendication 13, dans lequel un adhésif conducteur est pré-appliqué sur soit l'une soit les deux parmi les première et seconde paires de surfaces de contact.

15. Dispositif selon la revendication 14, dans lequel une languette de protection amovible est positionnée pour recouvrir l'adhésif sur les surfaces de contact de manière à empêcher toute contamination de celles-ci avant l'utilisation.

16. Dispositif selon l'une quelconque des revendications 13 à 15, dans lequel les surfaces de contact des première ou seconde parties ont une surface totale égale ou supérieure à 1 millimètre carré.

17. Dispositif selon l'une quelconque des revendications 1 à 16, dans lequel le circuit électrique comprend l'une ou une combinaison parmi :
• une source d'alimentation électrique à des fins d'alimentation du circuit ;
• une unité de traitement à des fins de réception d'informations en provenance du dispositif de commande, du dispositif de surveillance ou du dispositif de détection sur le paramètre faisant l'objet d'une surveillance, d'une détection ou d'une commande et à des fins de comparaison des informations se rapportant au paramètre par rapport à une valeur présélectionnée ou un ensemble de valeurs du paramètre ;
• une interface de dispositif de commande pour permettre le fonctionnement marche et arrêt de la source d'alimentation ou la commande d'une autre fonction opérationnelle du dispositif ;
• un système de sortie permettant d'envoyer des signaux traités ou non traités à des fins de signalisation quand les informations se rapportant au paramètre faisant l'objet d'une commande, d'une surveillance ou d'une détection sont égales, supérieures ou inférieures à une valeur présélectionnée du paramètre faisant l'objet d'une surveillance, d'une détection ou d'une commande.

18. Dispositif selon la revendication 17, dans lequel l'unité de traitement est une puce de traitement ou un microcontrôleur.

19. Dispositif selon la revendication 17, dans lequel l'interface de commande est un commutateur.

20. Dispositif selon l'une quelconque des revendications 17 à 19, dans lequel le système de sortie comprend l'un ou une combinaison parmi : un émetteur qui émet un signal à un récepteur ; ou un dispositif indicateur qui émet de la lumière et/ou du son.

21. Dispositif selon l'une quelconque des revendications 18 à 20, dans lequel le système de sortie émet un signal à une station centralisée ou un administrateur de soins qui peut avoir à sa charge un ou un certain nombre de patients.

22. Dispositif selon l'une quelconque des revendications 1 à 19, dans lequel le substrat flexible sur lequel la première partie du circuit est portée est incorporé dans un vêtement ou un textile destiné à réaliser un vêtement, ou constitue une partie de celui-ci.

23. Dispositif selon la revendication 22, dans lequel le vêtement est une couche jetable, une serviette sanitaire, un produit sanitaire d'incontinence ou un pansement, et le paramètre faisant l'objet d'une surveillance, d'une détection ou d'une commande est l'humidité et la première partie du circuit portée par le substrat flexible constitue une partie du vêtement et peut être jetée et la seconde partie du circuit peut être réutilisée en la retirant du vêtement utilisé et en la connectant à un nouveau vêtement.

24. Dispositif qui peut être porté par une personne ou installé sur un appareil à des fins de surveillance, de détection ou de commande d'un ou de plusieurs paramètres tels, mais de façon non limitative, l'humidité, la température, le rythme cardiaque, le rythme respiratoire, l'effort ; le mouvement, l'intensité lumineuse, le son, les forces de pression ou d'impact, ou une fonction électrique, dans lequel le dispositif comprend :
a) un substrat flexible (10) qui peut être porté par une personne ou être attaché sur un appareil, dans lequel le substrat flexible (10) a besoin d'être jeté ou remplacé par un nouveau substrat flexible ou a besoin d'un entretien qui empêche une utilisation continue du substrat flexible (10) ; et
b) une première partie (12) d'un circuit électrique qui est en mesure d'être connectée à une seconde partie (16) du circuit électrique, dans lequel la première partie (12) est attachée au substrat flexible (10) et la première partie (12) comprend une paire de surfaces de contact (13) à des fins de connexion avec une paire de surfaces de contact (14) de la seconde partie (16) qui coopèrent ;
**caractérisé en ce qu'**un adhésif conducteur est pré-appliqué sur les surfaces de contact (13) de la première partie (12) de telle sorte que les première et seconde parties (12, 16) peuvent être connectées ensemble pour former une connexion électrique entre elles et par la suite déconnectées pour permettre à la première partie du circuit d'être jetée selon les besoins avec le substrat.

25. Dispositif selon la revendication 24, dans lequel la première partie du circuit est en mesure de fléchir de telle sorte qu'à la fois le substrat flexible et la première partie du circuit sont en mesure d'être fléchis.

26. Dispositif selon la revendication 24 ou la revendication 25, dans lequel la première partie du circuit comprend un circuit imprimé qui est formé sur le substrat flexible ou qui est intégré dans celui-ci.

27. Dispositif selon l'une quelconque des revendications 24 à 26, dans lequel la première partie du circuit comprend un matériau conducteur qui est sur le substrat flexible ou qui est intégré dans celui-ci.

28. Dispositif selon la revendication 26, dans lequel le matériau conducteur est un filament ou fil qui a été tissé, tricoté ou incorporé dans le substrat flexible au moyen d'une structure en couches.

29. Dispositif selon l'une quelconque des revendications 24 à 28, dans lequel avant la connexion des première et seconde parties une languette de protection amovible est positionnée pour recouvrir l'adhésif sur les surfaces de contact de manière à empêcher toute contamination de celles-ci avant l'utilisation.

30. Dispositif selon l'une quelconque des revendications 24 à 29, dans lequel les surfaces de contact de la première partie ont une surface totale égale ou supérieure à 1 millimètre carré.

31. Dispositif selon l'une quelconque des revendications 24 à 30, dans lequel le substrat flexible sur lequel la première partie du circuit est portée est incorporé dans un vêtement ou un textile destiné à réaliser un vêtement, ou constitue une partie de celui-ci.

32. Dispositif selon la revendication 31, dans lequel le vêtement est une couche jetable, une serviette sanitaire, un produit sanitaire d'incontinence ou un pansement, et le paramètre faisant l'objet d'une surveillance, d'une détection ou d'une commande est l'humidité et la première partie du circuit portée par le substrat flexible constitue une partie du vêtement et peut être jetée et la seconde partie du circuit peut être réutilisée en la retirant du vêtement utilisé et en la connectant à un nouveau vêtement.
